# EUROPEAN PATENT SPECIFICATION

(11) **EP 2 390 810 B1**
(45) Date of publication and mention of the grant of the patent: **16.10.2019**
(21) Application number: 11167492.5
(22) Date of filing: 25.05.2011
(51) Int. Cl.: G16B 10/00, G16B 40/30, G16B 30/10

(54) **Taxonomic classification of metagenomic sequences**
Taxonomische Klassifizierung von metagenomen Sequenzen
Classification taxinomique de séquences métagénomiques

(30) Priority: 26.05.2010 IN 1628MU2010
(43) Date of publication of application: 30.11.2011
(73) Proprietor: Tata Consultancy Services Limited, 400021 Mumbay (IN)
(72) Inventor: Mande, Sharmila S., Hyderabad 500081 Andhra Pradesh (IN); Haque, Mohammed Monzoorul, Hyderabad 500081 Andhra Pradesh (IN); Ghosh, Tarini Shankar, Hyderabad 500081 Andhra Pradesh (IN); Singh, Nitin Kumar, Sultanpur 228141 Uttar Pradesh (IN)
(74) Representative: Zacco Sweden AB

(56) References cited:
- ARTHUR BRADY ET AL: "Phymm and PhymmBL: metagenomic phylogenetic classification with interpolated Markov models", NATURE METHODS, vol. 6, no. 9, 1 September 2009 (2009-09-01), pages 673-676, XP55033321, ISSN: 1548-7091, DOI: 10.1038/nmeth.1358
- M. H. MOHAMMED ET AL: "SPHINX--an algorithm for taxonomic binning of metagenomic sequences", BIOINFORMATICS, vol. 27, no. 1, 28 October 2010 (2010-10-28), pages 22-30, XP55033068, ISSN: 1367-4803, DOI: 10.1093/bioinformatics/btq608
- ALICE CAROLYN MCHARDY ET AL: "Accurate phylogenetic classification of variable-length DNA fragments", NATURE METHODS, vol. 4, no. 1, 1 January 2007 (2007-01-01) , pages 63-72, XP55033074, ISSN: 1548-7091, DOI: 10.1038/nmeth976
- DIAZ NARYTTZA N ET AL: "TACOA â Taxonomic classification of environmental genomic fragments using a kernelized nearest neighbor approach", BMC BIOINFORMATICS, BIOMED CENTRAL, LONDON, GB, vol. 10, no. 1, 11 February 2009 (2009-02-11), page 56, XP021047319, ISSN: 1471-2105, DOI: 10.1186/1471-2105-10-56
- M. MONZOORUL HAQUE ET AL: "SOrt-ITEMS: Sequence orthology based approach for improved taxonomic estimation of metagenomic sequences", BIOINFORMATICS, vol. 25, no. 14, 13 May 2009 (2009-05-13), pages 1722-1730, XP55033242, ISSN: 1367-4803, DOI: 10.1093/bioinformatics/btp317

## Description

### TECHNICAL FIELD

The present subject matter relates, in general, to the field of metagenomics and, in particular, taxonomic classification of metagenomic sequences.

### BACKGROUND

The study of genetic material recovered directly from an environmental sample, by sequencing the genetic material, thereby bypassing isolation and cultivation step is referred to as metagenomics. Metagenomics provides information pertaining to taxonomic diversity and physiology of various organisms present in an environmental sample.

In order to gather information pertaining to taxonomic classification of an environment sample, the genetic material obtained directly from the environmental sample is sequenced in to a plurality of sequences, called metagenomic sequences. Each of these metagenomic sequences are then classified or cataloged into various taxonomic groups, such as kingdom, phylum, class, order, family, genus, or species. This whole process of classifying metagenomic sequences is called taxonomic classification or binning.

Taxonomic classification of metagenomic sequences, as the one mentioned above, helps reconstructing the microbial composition of the environmental sample. It also provides information regarding evolutionary history and previously unrecognized physiological abilities of microbial communities specialized to live in a given environmental niche. Taxonomic classification not only catalogs known organisms, but also classifies new organisms to corresponding taxonomic groups for subsequent analyses. Precise taxonomic classification of metagenomic sequences is important since wrongly classified sequences may affect the accuracy of several downstream analyses, for example, sequence assembly, gene prediction, and functional annotation.

Researchers typically employ a variety of taxonomic classification techniques to classify metagenomic sequences. Conventional taxonomic classification techniques associate a sequence to a taxon if a feature of the sequence, such as composition or sequence similarity, is similar to reference sequences belonging to that taxon. However, such taxonomic classification techniques are either time consuming or prevent users from assessing the taxonomic diversity of environmental samples at appropriate taxonomic levels.

### SUMMARY

This summary is provided to introduce concepts related to taxonomic classification of metagenomic sequences, which are further described below in the detailed description. This summary is not intended to identify essential features of the claimed subject matter nor is it intended for use in determining or limiting the scope of the claimed subject matter.

Method(s) and a system(s) for taxonomic classification of a query sequence, such as a metagenomic sequence, are described herein. In one implementation, a target cluster, from amongst a plurality of reference clusters, corresponding to the query sequence is selected. The target cluster may be selected based on a composition based analysis. Based on the target cluster, a similarity based analysis of the query sequence is performed with respect to sequences already stored in the target cluster. A taxon from the target cluster corresponding to the query sequence is identified.

### BRIEF DESCRIPTION OF THE DRAWINGS

The detailed description is described with reference to the accompanying figures. In the figures, the left-most digit(s) of a reference number identifies the figure in which the reference number first appears. The same numbers are used throughout the drawings to reference like features and components.
Fig. 1 illustrates an exemplary system for taxonomic classification of a query sequence, in accordance with an embodiment of the present subject matter.
Fig. 2 illustrates an exemplary method for taxonomic classification of a query sequence, in accordance with an implementation of the present subject matter
Fig 3 illustrates an exemplary method to classify reference sequences into reference clusters, in accordance with an implementation of the present subject matter.

### DETAILED DESCRIPTION

Typically, genetic material extracted directly from an environmental sample, i.e. metagenome, comprises a mixture of nucleic acids originating from different organisms present in that environment. The genetic material is sequenced to generate a plurality of metagenomic sequences, which are subsequently analyzed for estimating taxonomic diversity of the environmental sample. For the purposes of discussion, metagenomic sequences, or 'metagenomic reads', that are to be analyzed may be interchangeably referred to as query sequences.

A variety of taxonomic classification techniques have been used for the classification of query sequences derived from various organisms present in a given an environmental sample into their corresponding taxonomic groups. Conventional taxonomic classification techniques assign a taxon to a query sequence if features of the query sequence are similar to features of reference sequences belonging to that taxon. The extent to which the features of the query sequence are similar to the features of the reference sequences depends on the heuristics that the taxonomic classification technique uses, which, in turn, determines accuracy and specificity of the taxonomic classification technique.

Higher level taxa, for example, taxa at the level of root, cellular organisms, and super-kingdom may be referred to as non-specific taxa, while lower level taxa, for example, taxa at the level of phylum, class, order, family, genus and below may be referred to as specific taxa. Accordingly, assignment of a query sequence to a specific taxon increases the specificity of the taxonomic classification technique. Conversely, assignment of a query sequence to a non-specific taxon decreases the specificity of the taxonomic classification technique.

One class of the conventional taxonomic classification techniques includes composition-based techniques, such as described in Phylopythia (McHardy et al., Accurate phylogenetic classification of variable length DNA fragments. Nature Methods, Volume 4, pages 63-72, 2007), TACOA (Diaz et al., TACOA-Taxonomic classification of environmental genomic fragments using a kernelized nearest neighbor approach, BMC Bioinformatics, Volume 10, page 56, 2009), and PhymmBL (Brady et al., Phymm and PhymmBL: Metagenomic phylogenetic classification with interpolated Markov models, Nature Methods, Volume 6, pages 673-676, 2009). These techniques classify a query sequence based on similarity of compositional characteristics. The compositional characteristics may include, for example, guanine-cytosine (GC) content, oligonucleotide frequencies, etc. The GC content is the percentage of nucleobases in the query sequence, which are either guanine or cytosine, and the oligonucleotide frequency is the number of occurrences of an oligonucleotide of a given length in a query sequence.

The conventional composition-based techniques may not be robust enough to identify a taxon corresponding to a query sequence with a short sequence length, for example, less than 1000 base pairs. Such composition based techniques, usually, assign a majority of query sequences to non-specific taxa, for example, taxa at the level of root, cellular organisms, super-kingdom.

Another class of the taxonomic classification techniques includes similarity-based techniques. The similarity-based classification techniques seek to identify a taxon corresponding to a query sequence by comparing the query sequence with every individual reference sequence stored in a database. For the purpose of comparing, the similarity based techniques may use alignment tools, such as Basic Local Alignment Search Tool (BLAST). Based on the comparison, information pertaining to alignment of the query sequence and the reference sequences is obtained. Accordingly, reference sequences homologous to the query sequence, i.e., suggesting a common ancestry or evolutionary origin with the query sequence, are analyzed to identify a taxon corresponding to the query sequence.

Generally, similarity-based techniques have greater accuracy and specificity as compared to the composition-based techniques. However, the similarity-based techniques involve substantial computational time and resources for aligning a query sequence against each of the reference sequences stored in a database, such as a non-redundant (nr) database that contains more than 9 million sequences.

Thus, the conventional taxonomic classification techniques as mentioned above are either not efficient in terms of accuracy and specificity or involve considerable computational time and resources, thereby having limited application.

To this end, methods and systems for taxonomic classification of query sequences is described herein. The taxonomic classification is with respect to a reference database having a plurality of reference clusters, which are created by grouping a plurality of reference sequences. The reference sequences are grouped into corresponding reference clusters based on their compositional characteristics. In one implementation, the reference sequences may be nucleic acid reference sequences, which may be clustered based on nucleotide compositional characteristics, such as tetranucleotide frequency.

Alternatively, other nucleotide compositional characteristics, for example, GC content and or any other oligonucleotide frequency, may also be used. Further, each of the reference clusters may contain amino acid reference sequences corresponding to the nucleic acid reference sequences. In another implementation, the reference sequences may be amino acid reference sequences, which may be clustered using amino acid compositional characteristics, such as oligo-peptide frequencies.

In one implementation, a cluster centroid is computed for each of the reference clusters. Subsequently, the cluster centroids are tagged to their corresponding reference clusters. At least one target cluster, from amongst the plurality of the reference clusters, corresponding to the query sequence may be selected based on a composition-based analysis of the query sequence. The composition-based analysis computes a distance between a query vector corresponding to the query sequence and the cluster centroid of each of the reference clusters. Further, the target cluster is selected based on the computed distances.

Once the target cluster is selected, a similarity-based analysis of the query sequence is performed with respect to reference sequences in the target cluster. In one implementation, the similarity-based analysis involves performing a six frame translation of the query sequence into corresponding amino acid query sequences and comparison of the translated query sequence with respect to the amino acid reference sequences of the target cluster. Based on the comparison, a taxon corresponding to the query sequence is identified and assigned to the query sequence.

Although, the similarity-based analysis has been explained with respect to amino acid sequences, it will be understood that the similarity-based analysis may also be performed with respect to the nucleic acid sequences in a similar manner. Since the similarity based analysis is performed only with respect to reference sequences in the target cluster, the search space for the similarity based analysis is considerably reduced, thereby improving the efficiency of the taxonomic classification, in terms of computational time and resources. Further, as will be shown using various test case scenarios, the accuracy and specificity of the present taxonomic classification technique is comparable to conventional similarity-based techniques.

The reference database may include coding or non-coding reference sequences or references having both coding and non-coding regions. As is known in the art, a coding sequence is a gene sequence that codes for a protein and its antithesis is a non-coding sequence. Likewise, the query sequence may also be a coding or non-coding sequence, or a sequence having a combination of both coding and non-coding regions. Further, the reference database may include reference sequences in the form of nucleic acid sequences, hereinafter referred to as the nucleic acid reference sequences. Additionally or alternately, the reference database may include the reference sequences in the form of amino acid sequences, hereinafter referred to as the amino acid reference sequences. The nucleic acid reference sequences differ from the amino acid reference sequences in that the nucleic acid sequences include nucleobases, for example, cytosine, guanine, adenine and thymine/uracil, arranged in a sequence, while the amino acid reference sequences comprise amino acids arranged in a sequence.

While aspects of described systems and methods for the taxonomic classification of the metagenomic sequences can be implemented in any number of different computing systems, environments, and/or configurations, the embodiments are described in the context of the following exemplary system(s).

### EXEMPLARY SYSTEMS

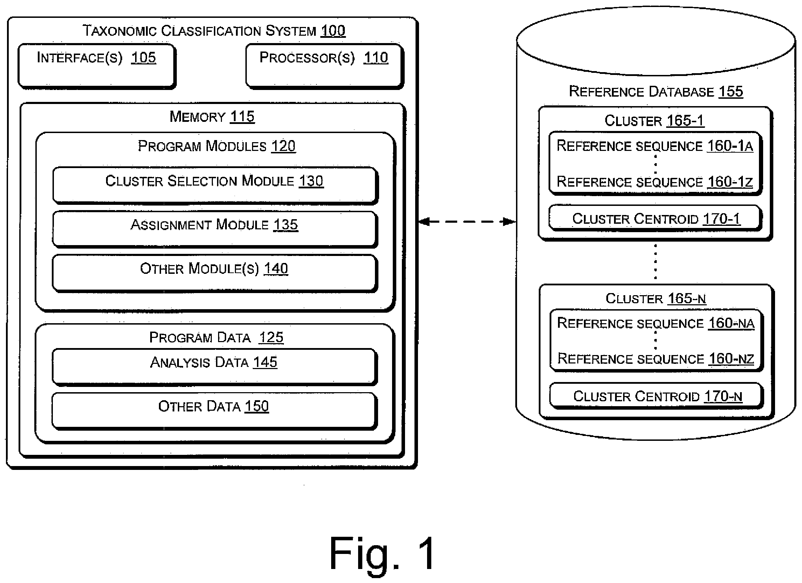

Fig. 1 illustrates an exemplary taxonomic classification system 100, according to an implementation of the present subject matter. The taxonomic classification system 100 can be implemented in systems that include, but are not limited to, desktop computers, hand-held devices, multiprocessor systems, personal digital assistants (PDAs), laptops, network computers, cloud servers, minicomputers, mainframe computers, and the like. In one implementation, the taxonomic classification system 100 includes interface(s) 105, one or more processor(s) 110, and a memory 115 coupled to the processor(s) 110.

The interfaces 105 may include a variety of software and hardware interfaces, for example, interfaces for peripheral device(s), such as a keyboard, a mouse, an external memory, and a printer. Further, the interfaces 105 may enable the taxonomic classification system 100 to communicate with other computing systems, such as web servers and external databases. The interfaces 105 can facilitate multiple communications within a wide variety of networks and protocol types, including wired networks, for example local area network (LAN), cable, etc., and wireless networks such as Wireless LAN (WLAN), cellular, or satellite. For the purpose, the interfaces 105 may include one or more ports for connecting a number of computing systems with one another or to another server computer.

The processor 110 can be a single processing unit or a number of units, all of which could include multiple computing units. The processor 110 may be implemented as one or more microprocessors, microcomputers, microcontrollers, digital signal processors, central processing units, state machines, logic circuitries, and/or any devices that manipulate signals based on operational instructions. Among other capabilities, the processor 110 is configured to fetch and execute computer-readable instructions and data stored in the memory 115.

The memory 115 may include any computer-readable medium known in the art including, for example, volatile memory such as static random access memory (SRAM) and dynamic random access memory (DRAM), and/or non-volatile memory, such as read only memory (ROM), erasable programmable ROM, flash memories, hard disks, optical disks, and magnetic tapes. The memory 115 also includes program module(s) 120 and program data 125.

The program modules 120, amongst other things, include routines, programs, objects, components, data structures, etc., which perform particular tasks or implement particular abstract data types. The program modules 120 further include, for example, a cluster selection module 130, an assignment module 135, and other module(s) 140. The other modules 140 may include programs that supplement applications on the taxonomic classification system 100, for example, programs in the operating system. On the other hand, the program data 125 serves, amongst other things, as a repository for storing data processed, received, and generated by one or more of the program modules 120. The program data 125 includes, for example, analysis data 145 and other data 150. The other data 150 includes data generated as a result of the execution of one or more modules in the other modules 140.

In one example, the taxonomic classification system 100 is associated with a reference database 155. The reference database 155 either can be external or internal to the taxonomic classification system 100. The reference database 155 includes a plurality of reference sequences 160-1A...160-NZ, hereinafter collectively referred to as reference sequences 160. The reference sequences 160 can be classified into a plurality of reference clusters 165-1... 165-N, hereinafter collectively referred to as reference clusters 165. Subsequent to classification of the reference sequences 160, the reference cluster 165-1 includes reference sequences 160-1A to 160-1Z, the reference cluster 165-2 includes reference sequences 160-2A to 160-2Z, and so on.

In one implementation, the reference sequences 160 are in the form of nucleic acid sequences. Additionally or alternately, the reference database 155 may include amino acid reference sequences corresponding to the reference sequences 160. Further, the reference sequences 160 in the reference database 155 may contain coding sequences or non-coding sequences or sequences having both coding and non-coding regions.

In one example, the reference database 155 may be a pre-configured database, which includes reference sequences 160 grouped into reference clusters 165 based on one or more compositional characteristics of the reference sequences 160. Alternatively, the cluster selection module 130 may categorize the reference sequences 160 into the reference clusters 165 based on the compositional characteristics of the reference sequences 160. Although, the clustering of the reference sequences 160 is explained in considerable detail with reference to tetra-nucleotide frequency as a compositional characteristic, it will be appreciated that other compositional characteristics, for example, GC content or any other oligonucleotide frequencies, may also be used for clustering of the reference sequences 160.

In one example, the cluster selection module 130 computes frequencies of all possible tetra-nucleotides in each of the reference sequences 160 and, accordingly, generates a reference vector corresponding to each of the reference sequences 160. The reference vectors may be stored as 256-dimensional vectors. Subsequently, the reference sequences 160, based on the corresponding reference vectors, may be classified into the reference clusters 165 using conventional clustering techniques, such as k-means clustering technique in which n number of observations are partitioned into k number of clusters such that each observation belongs to a cluster with the nearest mean.

In an example, in order to form reference clusters 165, the cluster selection module 130 selects a predetermined number of reference clusters and randomly tags the reference clusters 165 with a plurality of cluster centroids 170-1...170-N, hereinafter collectively referred to cluster centroids 170. For each of the reference vectors, a cluster centroid closest to a reference sequence is determined, and the reference sequence is moved to the reference cluster corresponding to the closest cluster centroid..In this way, reference sequences 165 with similar compositional characteristics are moved to same reference cluster.

The closest reference cluster for a reference sequence may be determined based on a distance between corresponding reference vector and each of the cluster centroids 170. The distance, for example, may be measured in terms of Euclidean metrics or non-Euclidean distance metrics, for example, Manhattan distance (L1 norm). Further, if the reference sequence under consideration is moved to a reference cluster, the clusters centroids 170 are computed again. A cluster centroid represents a mean value of the reference vectors corresponding to the reference sequences present in a reference cluster. In one example, the process of forming the reference clusters 165 can be performed repeatedly till the reference clusters 165 become stable or some maximum number of iterations have been performed. In one implementation, the cluster centroids 170 may be tagged to the corresponding reference clusters 165.

Additionally or alternatively, the cluster selection module 130 may translate the reference sequences 160 into corresponding amino acid reference sequences and store the amino acid reference sequences in their respective reference clusters 165.

In one example, the taxonomic classification system 100 accepts an input file in FASTA format. The input file may contain either single or multiple query sequences. The cluster selection module 130 selects one or more target cluster(s), from the reference clusters 165, corresponding to the query sequence. In one implementation, the selection of the one or more target clusters is based on composition of the query sequence. The cluster selection module 130, to select a target cluster, may initially generate a query vector corresponding to the query sequence. The query vector may be generated based on one or more compositional characteristics, such as the frequencies of all possible tetra-nucleotides in the query sequence. It will be understood that compositional characteristics used for generating a query vector would be similar to the compositional characteristics used for generating a reference vector.

Subsequently, distances, such as Manhattan distance (L1 norm), between the query vector and each of the cluster centroids 170 are evaluated. The cluster selection module 130 selects a cluster, say cluster 165-1, having a minimum distance to the query vector, as the target cluster. For the purpose of explanation, and not as a limitation, the cluster 165-1 will be hereinafter referred to as the target cluster 165-1.

Subsequent to selection of the target cluster 165-1, the assignment module 135 performs a similarity-based analysis of the query sequence with respect to reference sequences 160-1A to 160-1Z in the target cluster 165-1. The similarity-based analysis may be performed, for example, using Basic Local Alignment Search Tool (BLAST). In one example, the degree of homology between the query sequence and the reference sequences 160-1A to 160-1Z present in the target cluster 165-1, which are obtained using the similarity based analysis, may be used for identifying and assigning a taxon corresponding to the query sequence. The degree of homology may be based on the quality of alignment between the query sequence and the reference sequences 160-1A to 160-1Z in the target cluster 165-1. For example, a higher degree of homology between a query sequence and the reference sequences 160-1A to 160-1Z may result in the assignment of the query sequence to a specific taxon. Conversely, a lower degree of homology between a query sequence and the reference sequences 160 may result in the assignment of the query sequence to a non-specific taxon.

In one example, the assignment module 135 may translate the query sequence into corresponding amino acid query sequences for the similarity-based analysis. In said implementation, the assignment module 135 compares the translated query sequence with each of the amino acid reference sequences in the target cluster 165-1. Alternatively, the similarity-based analysis may be performed for the query sequence, which is in the form of nucleic acid sequence, with the nucleic acid reference sequences in the target cluster 165-1.

The result of the similarity based analysis may be stored in the analysis data 145. The analysis data 145 may include information pertaining to taxon of each of the reference sequences 160, the degree of homology between the query sequence and the reference sequences 160, etc. As previously mentioned, the degree of homology may be computed based on the quality of alignment and accordingly may be computed based on one or more alignment parameters. Alignment parameters include bit-score, percentage of identity and the percentage of positives between the query sequence and each of the reference sequences 160.

While 'identities' indicate the percentage of identical residues in the alignment, 'positives' correspond to the percentage of residues in the alignment for which the alignment scores have positive values. The bit score, on the other hand, gives an indication of how good the alignment is. The higher the score, the better is the alignment. In other words, the bit score takes into account the alignment of similar or identical residues, as well as any gaps introduced in aligning the sequences.

The degree of homology may be used to identify those reference sequences in the target cluster 165-1 that exhibit significant similarity with the query sequence, thus indicating a common taxonomic origin. Based on the analysis data 145, the assignment module 135 identifies and assigns a taxon corresponding to the query sequence. In one example, the assignment module 135 identifies and assigns the query sequence to a taxon at an appropriate taxonomic level. The selection of the taxonomic level is based on the degree of homology between the query sequence and the reference sequences 160 in the target cluster 165-1.

For example, a query sequence is assigned to a taxon at a specific taxonomic level, such as genus, if the degree of homology between the query sequence and the reference sequences in the target cluster 165-1 are greater than a predetermined threshold value. In one implementation, the assignment module 135 may utilize predetermined threshold values as discussed in 'SOrt-ITEMS' (Monzoorul Haque et al., SOrt-ITEMS : Sequence Orthology based approach for binning and Improved Taxonomic Estimation of Metagenomic Sequences, Bioinformatics, Volume 25(14), pages 1722-1730, 2009).

The provision of having the reference database 155 including the reference sequences 160 classified into plurality of reference clusters 165 and performing the similarity based analysis in the target cluster 165-1, provides for a reduction in computing time and resources. Since, the similarity-based analysis is performed only against the reference sequences 160 in the target cluster 165-1, instead of the entire reference database 155, the search space for the similarity-based analysis is substantially reduced. The reduction in the search space provides for substantial reduction of computational time and, at the same time, maintains accuracy and specificity of the taxonomic classification system 100.

### VALIDATION AND RESULTS

The results of present taxonomic classification have been validated using a "leave one clade out" strategy. In this strategy, reference sequences such as the reference sequences 160, belonging to an entire clade, corresponding to a plurality of taxa, are removed from the database such as the reference database 155. The removed clade may correspond to taxa belonging to one genus, family, order, class, or phylum. Query sequences derived from the taxa belonging to the removed clade are analyzed against this modified database, which acts as a reference database 155. Such a strategy is intended to closely mimic a typical metagenomic scenario wherein a majority of query sequences belong to hitherto unknown or new clades.

For the purpose of validation, a modified reference database was created, wherein coding sequences corresponding to 300 randomly selected genomes from 952 genomes were removed prior to creating clusters in the reference database 155. Tracing of the taxonomic lineage of these 300 genomes revealed complete removal of certain clades from the reference database. Subsequent to creation of clusters in the modified reference database, the present taxonomic classification techniques, illustrated as embodiments of the present subject matter, were validated using 1,40,000 query sequences of varying lengths, which were generated using MetaSim (Daniel et al., MetaSim-A Sequencing Simulator for Genomics and Metagenomics, PLoS ONE, Volume 3 (10) e3373, 2008).

Further, to mimic a typical metagenomic scenario, about 1,32,000 (94.2%) query sequences were derived from organisms belonging to those clades that were removed while creating the modified reference database (which now acts as the reference database 155). Depending on the lengths of the query sequences, the query sequences were divided into four validation data sets, termed as, Sanger data set, 454-400 data set, 454-250 data set, and 454-100 data set. Each of these data sets contained 35,000 query sequences. The query sequences constituting these four data sets simulated typical sequence lengths and error models obtained from commonly used sequencing techniques.

For example, query sequences constituting the Sanger data set had read length or sequence length centered around 800 base pairs, 454-400 data set had sequence length centered around 400 base pairs, 454-250 data set had sequence length centered around 250 base pairs, and 454-100 data set had sequence length centered around 100 base pairs. Further, the Sanger data set simulated reads or sequences obtained using Sanger sequencing technology;, 454-400 data set simulated sequences obtained using 454-GS-FLX-Titanium sequencing technology; 454-250 data set simulated sequences obtained using 454-GS-FLX-Standard sequencing technology, and,454-100 data set simulated sequences obtained using Roche-454-GS20 sequencing technology.

As also said earlier, assignment of query sequences to taxa at the level of phylum or below is considered specific, while those above the level of phylum are considered non-specific. Assignment of a query sequence to a taxon that either corresponds to its source organism or to a taxon that lies in the path from the root to the taxon corresponding to the source organism of the query sequence, may be referred to as "correct". Likewise, assignment of a query sequence to a taxon that does not lie in the path from the root to the taxon corresponding to the source organism, may be referred to as "wrong". Further, those query sequences that display weak homology with the reference sequences in the target cluster of the modified reference database, with alignment parameters falling below predefined thresholds, are classified as "unassigned". Furthermore, the reference database used for evaluating the present taxonomic classification technique was in a clustered format, i.e., the reference database contained reference clusters (such as the reference clusters 165). On the other hand, a conventional un-clustered form of the same reference database was used for evaluating the conventional similarity based and composition based techniques.

Following tables 1a, 1b, 1c, and 1d, collectively referred to as Table 1, depict comparison of the taxonomic assignment of query sequences obtained using the present taxonomic classification technique, according to one implementation of the present subject matter, and a conventional similarity-based taxonomic classification technique, for example, SOrt-ITEMS, for 454-100 data set, 454-250 data set, 454-400 data set, and Sanger data set, respectively. The taxonomic assignments obtained using both the present classification technique and the conventional similarity-based classification technique are with respect to a reference database containing coding sequences.

| Table 1a | | | | |
|---|---|---|---|---|
| 454-100 Data Set (Query Sequence Length: 100 base pairs) | | | | |
| Reference Database : Coding Sequences from 652 prokaryotic organisms | | | | |
| Total Query Sequences : 35,000 | | | | |
| Taxonomic Level | Assignments using Present Classification Technique | | Assignments using Conventional Similarity based Technique | |
| | Total Number | Percentage | Total Number | Percentage |
| Non Specific | 4728 | 13.51 | 1679 | 4.8 |
| Phylum | 6122 | 17.49 | 3932 | 11.23 |
| Class | 78 | 0.22 | 1078 | 3.08 |
| Order | 0 | 4.46 | 0 | 0 |
| Family | 1560 | 2.26 | 4017 | 11.48 |
| Genus & | 790 | 8.08 | 1849 | 5.28 |
| | | | | |
| Wrong | 2610 | 7.46 | 1900 | 5.43 |
| Unassigned | 19112 | 54.61 | 20545 | 58.7 |
| Specific | 8550 | 24.43 | 10876 | 31.07 |
| Total Correct | 13278 | 37.34 | 12555 | 35.87 |

| Table 1b | | | | |
|---|---|---|---|---|
| 454-250 Data Set (Query Sequence Length: 250 base pairs) | | | | |
| Reference Database : Coding Sequences from 652 prokaryotic organisms | | | | |
| Total Query Sequences : 35,000 | | | | |
| Taxonomic Level | Assignments using Present Classification Technique | | Assignments using Conventional Similarity based Technique | |
| | Total Number | Percentage | Total Number | Percentage |
| Non Specific | 4023 | 11.49 | 4816 | 13.76 |
| Phylum | 5214 | 14.9 | 3849 | 11 |
| Class | 1738 | 4.97 | 4734 | 13.53 |
| Order | 0 | 0 | 0 | 0 |
| Family | 3218 | 9.19 | 3922 | 11.21 |
| Genus & | 2430 | 6.94 | 2626 | 7.5 |
| | | | | |
| Wrong | 2450 | 7 | 2525 | 7.21 |
| Unassigned | 15927 | 45.51 | 12528 | 35.79 |
| Specific | 12600 | 36 | 15131 | 43.23 |
| Total Correct | 16623 | 47.49 | 19947 | 56.99 |

| Table 1c | | | | |
|---|---|---|---|---|
| 454-400 Data Set (Query Sequence Length: 400 base pairs) | | | | |
| Reference Database : Coding Sequences from 652 prokaryotic organisms | | | | |
| Total Query Sequences : 35,000 | | | | |
| Taxonomic Level | Assignments using Present Classification Technique | | Assignments using Conventional Similarity based Technique | |
| | Total Number | Percentage | Total Number | Percentage |
| Non Specific | 4793 | 13.69 | 5168 | 14.77 |
| Phylum | 7403 | 21.15 | 8384 | 23.95 |
| Class | 1646 | 4.7 | 3606 | 10.3 |
| Order | 0 | 0 | 0 | 0 |
| Family | 4184 | 11.95 | 5268 | 15.05 |
| Genus & below | 3111 | 8.89 | 2562 | 7.32 |
| | | | | |
| Wrong | 4211 | 12.03 | 3477 | 9.93 |
| Unassigned | 9652 | 27.58 | 6535 | 18.67 |
| Specific | 16344 | 46.7 | 19820 | 56.63 |
| Total Correct | 21137 | 60.39 | 24988 | 71.39 |

| Table 1d | | | | |
|---|---|---|---|---|
| Sanger Data Set (Query Sequence Length: 800 base pairs) | | | | |
| Reference Database : Coding Sequences from 652 prokaryotic organisms | | | | |
| Total Query Sequences : 35,000 | | | | |
| Taxonomic Level | Assignments using Present Classification Technique | | Assignments using Conventional Similarity based Technique | |
| | Total Number | Percentage | Total Number | Percentage |
| Non Specific | 5055 | 14.44 | 5613 | 16.04 |
| Phylum | 8182 | 23.38 | 9845 | 28.13 |
| Class | 4215 | 12.04 | 6164 | 17.61 |
| Order | 0 | 0 | 0 | 0 |
| Family | 4290 | 12.26 | 4410 | 12.6 |
| Genus & | 2829 | 8.08 | 2078 | 5.94 |
| | | | | |
| Wrong | 1858 | 5.31 | 1293 | 3.69 |
| Unassigned | 8571 | 24.49 | 5597 | 15.99 |
| Specific | 19516 | 55.76 | 22497 | 64.28 |
| Total Correct | 24571 | 70.2 | 28110 | 80.31 |

Similarly, tables 2a, 2b, 2c, and 2d, collectively referred to as Table 2, depict comparison of taxonomic assignment of query sequences obtained using the present taxonomic classification technique with respect to a conventional composition based taxonomic classification technique, for example TACOA, for 454-100 data set, 454-250 data set, 454-400 data set, and Sanger data set respectively. The taxonomic assignments obtained using the present classification technique and the conventional composition based classification technique are also with respect to a reference database containing coding and non-coding sequences of about 652 prokaryotic organisms.

| Table 2a | | | | |
|---|---|---|---|---|
| 454-100 Data Set (Query Sequence Length: 100 base pairs) | | | | |
| Reference Database : Coding and Non-coding Sequences from 652 prokaryotic organisms | | | | |
| Total Query Sequences : 35,000 | | | | |
| Taxonomic Level | Assignments with Present Classification Technique | | Assignments with Conventional Composition based Technique | |
| | Total Number | Percentage | Total Number | Percentage |
| Non Specific | 7190 | 20.54 | 14253 | 40.72 |
| Phylum | 7835 | 22.39 | 926 | 2.65 |
| Class | 154 | 0.44 | 266 | 0.76 |
| Order | 124 | 0.35 | 157 | 0.45 |
| Family | 2159 | 6.17 | 0 | 0 |
| | | | | |
| Genus & below | 1491 | 4.26 | 166 | 0.47 |
| Wrong | 1824 | 5.21 | 11080 | 31.66 |
| Unassigned | 14223 | 40.64 | 8152 | 23.29 |
| Specific | 11763 | 33.61 | 1515 | 4.33 |
| Total Correct | 18953 | 54.15 | 15768 | 45.05 |

| Table 2b | | | | |
|---|---|---|---|---|
| 454-250 Data Set (Query Sequence Length: 250 base pairs) | | | | |
| Reference Database : Coding and non-coding Sequences from 652 prokaryotic organisms | | | | |
| Total Query Sequences : 35,000 | | | | |
| Taxonomic Level | Assignments with Present Classification Technique | | Assignments with Conventional Composition based Technique | |
| | Total Number | Percentage | Total Number | Percentage |
| Non Specific | 6028 | 17.22 | 16387 | 46.82 |
| Phylum | 5591 | 15.97 | 1560 | 4.46 |
| Class | 1477 | 4.22 | 382 | 1.09 |
| Order | 1109 | 3.17 | 180 | 0.51 |
| Family | 3052 | 8.72 | 0 | 0 |
| | | | | |
| Genus & | 2334 | 6.67 | 261 | 0.75 |
| Wrong | 1866 | 5.33 | 9795 | 27.99 |
| Unassigned | 13543 | 38.69 | 6435 | 18.39 |
| Specific | 13563 | 38.75 | 2383 | 6.81 |
| Total Correct | 19591 | 55.97 | 18770 | 53.63 |

| Table 2c | | | | |
|---|---|---|---|---|
| 454-400 Data Set (Query Sequence Length: 400 base pairs) | | | | |
| Reference Database : Coding and non-coding Sequences from 652 prokaryotic organisms | | | | |
| Total Query Sequences : 35,000 | | | | |
| Taxonomic Level | Assignments with Present Classification Technique | | Assignments with Conventional Composition based Technique | |
| | Total Number | Percentage | Total Number | Percentage |
| Non Specific | 5431 | 15.52 | 17617 | 50.33 |
| Phylum | 7940 | 22.69 | 1867 | 5.33 |
| Class | 1559 | 4.45 | 423 | 1.21 |
| Order | 389 | 1.11 | 236 | 0.67 |
| Family | 4372 | 12.49 | 0 | 0 |
| | | | | |
| Genus & below | 3053 | 8.72 | 418 | 1.19 |
| Wrong | 4327 | 12.36 | 8746 | 24.99 |
| Unassigned | 7929 | 22.65 | 5693 | 16.27 |
| Specific | 17313 | 49.47 | 2944 | 8.41 |
| Total Correct | 22744 | 64.98 | 20561 | 58.75 |

| Table 2d | | | | |
|---|---|---|---|---|
| Sanger Data Set (Query Sequence Length: 800 base pairs) | | | | |
| Reference Database : Coding and non-coding Sequences from 652 prokaryotic organisms | | | | |
| Total Query Sequences : 35,000 | | | | |
| Taxonomic Level | Assignments with Present Classification Technique | | Assignments with Conventional Composition based Technique | |
| | Total Number | Percentage | Total Number | Percentage |
| Non Specific | 5028 | 14.37 | 19915 | 56.9 |
| Phylum | 8925 | 25.5 | 2450 | 7 |
| Class | 5026 | 14.06 | 0 | 0 |
| Order | 157 | 0.45 | 252 | 0.72 |
| Family | 4102 | 11.72 | 0 | 0 |
| | | | | |
| Genus & below | 2484 | 7.1 | 70 | 0.2 |
| Wrong | 2572 | 7.35 | 7746 | 22.13 |
| Unassigned | 67026 | 19.16 | 4567 | 13.05 |
| Specific | 20694 | 59.13 | 2772 | 7.92 |
| Total Correct | 25722 | 73.49 | 22687 | 64.82 |

As can be seen from Table 1, both the present taxonomic classification technique and one of the conventional similarity based technique, such as SOrt-ITEMS, assign 76-80% of correct assignments at specific levels, thereby indicating that the specificity of the present taxonomic classification technique is comparable to the specificity of the conventional similarity based technique. Further, it may also be observed that the total number of correct assignments made by the present classification technique is comparable to the conventional similarity based technique, thus indicating that both the techniques have approximately the same classification accuracy.

From Table 2, one may observe that the percentage of the correct assignments by the present taxonomic classification technique is comparable to the conventional composition-based technique, such as TACOA. However, a majority (85-90%) of assignments made by TACOA are at non-specific taxonomic levels as compared to 19-37% by the present classification technique. This indicates higher specificity for the present classification technique .

Further, computational time taken by the present taxonomic classification technique is substantially lower than the conventional similarity-based technique, and the same is illustrated by way of table 3. Table 3 depicts average time taken for taxonomic classification of 10,000 query sequences using the present taxonomic classification technique and the conventional similarity based classification technique, SOrt-ITEMS. The database used for both the classification techniques was similar, as also mentioned earlier, and includes coding sequences from about 652 prokaryotic organisms. These time estimates were obtained using a desktop computer with 2.33 GHz central processing unit (CPU) with 2GB random access memory (RAM).

Table 3 shows that the present taxonomic classification technique achieves 15 to 20 fold reduction in the computational time over conventional similarity based classification technique, such as SOrt-ITEMS.

**Table 3**

| Reference Database : Coding Sequences of 652 prokaryotic organisms | | |
|---|---|---|
| Query Sequence Length (in base pairs) | Average time taken (in minutes) by Present Classification Technique | Average time taken (in minutes) by Conventional Similarity based Technique |
| 800 | 70 | 1431 |
| 400 | 40 | 773 |
| 250 | 28 | 425 |
| 100 | 18 | 270 |

The results of the present taxonomic classification technique have been validated using an exemplary query sequence from Burkholderia cenocepacia AU chromosome 1; 826823-827891, with a length of 1068 base pairs. A conventional similarity-based analysis was performed using BLASTx against all reference sequences in a reference database. In the present example, the reference database contained 2.1 million coding sequences derived from 952 prokaryotic organisms. An output of search performed by BLASTx was generated in approximately 60 seconds. An analysis of this output indicated that significant hits correspond to various species belonging to genus Burkholderia. Accordingly, the query sequence was assigned to genus Burkholderia.

The present taxonomic classification technique initially generated a query vector indicative of a oligonucleotide composition of the query sequence. The query vector was generated based on frequencies of all possible tetra-nucleotides in the query sequence. Further, a target cluster, from a plurality of reference clusters stored in a reference database, corresponding to the query vector was identified.

The reference database included 2.1 million coding sequences from 952 prokaryotic organisms, which were pre-clustered using compositional characteristics. The target cluster contained 4321 sequences and was identified based on a Manhattan distance between the query vector and cluster centroids of each of the clusters in the reference database. It was observed that a majority of reference sequences in the target cluster originated from genera belonging to family Burkholderiaceae.

Subsequently, using BLASTx, a similarity based analysis of the query sequence was performed against amino acid reference sequences corresponding to the reference sequences of the target cluster. An output of search performed by BLASTx in the target cluster was generated in approximately 3 seconds, which is twenty times less than the time taken by the conventional similarity-based technique. An analysis of this output indicated that significant hits corresponded to various species belonging to genus Burkholderia.

Since in the present taxonomic classification technique, the similarity-based analysis is performed with respect to the target cluster and not the entire database, approximately 20 fold reduction in computational time is achieved. Further, as it can be seen, both the present taxonomic classification technique and the conventional similarity based technique assign the query sequence to the genus Burkholderia. Hence, the accuracy and the specificity of the present taxonomic classification are comparable to the conventional similarity based techniques.

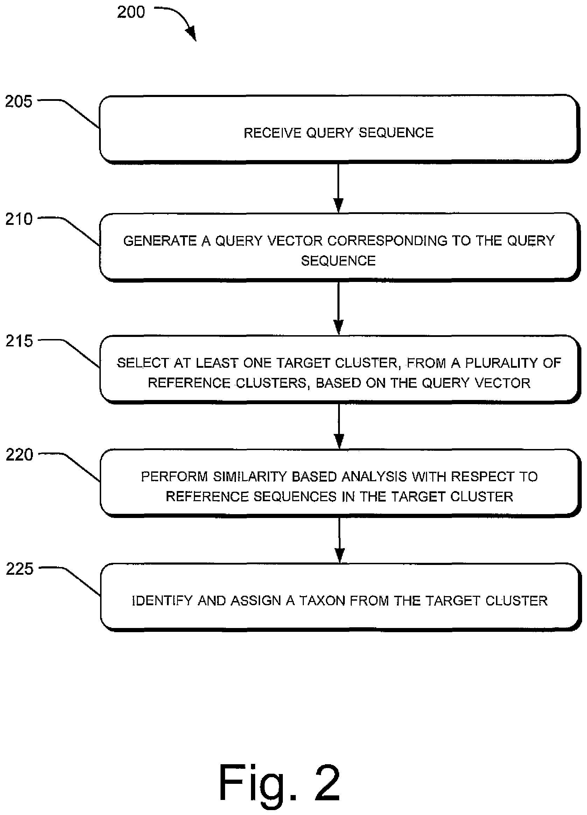

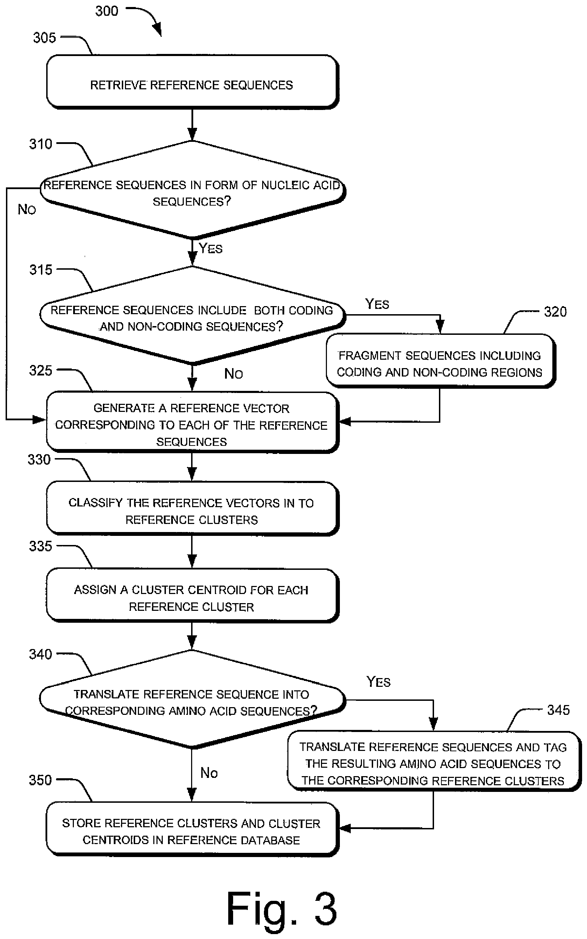

Fig. 2 illustrates an exemplary method 200 for taxonomic classification of a query sequence, while Fig. 3 illustrates an exemplary method 300 for classification of reference sequences into reference clusters, in accordance with one or more implementations of the present subject matter.

The exemplary methods may be described in the general context of computer executable instructions. Generally, computer executable instructions can include routines, programs, objects, components, data structures, procedures, modules, functions, etc., that perform particular functions or implement particular abstract data types. The methods may also be practiced in a distributed computing environment where functions are performed by remote processing devices that are linked through a communications network. In a distributed computing environment, computer executable instructions may be located in both local and remote computer storage media, including memory storage devices.

The order in which the methods are described is not intended to be construed as a limitation, and any number of the described method blocks can be combined in any order to implement the method, or an alternative method. Additionally, individual blocks may be deleted from the methods without departing from the spirit and scope of the subject matter described herein. Furthermore, the methods can be implemented in any suitable hardware, software, firmware, or combination thereof. The method is presently provided for a query sequence. It would be appreciated that the same method can also be implemented for a plurality of query sequences without deviating from the scope of the present subject matter.

At block 205, a query sequence, i.e. a metagenomic sequence, is received. The query sequence may be in the form of a nucleic acid sequence or an amino acid sequence. For example, the query sequence from a metagenome can be received by a cluster selection module, such as the cluster selection module 130.

At block 210, a query vector corresponding to the query sequence is generated, based on a composition based analysis of the query sequence. For example, the cluster selection module 130 on receiving the query sequence performs a composition based analysis to generate a query vector. The composition based analysis is performed based on compositional characteristics, such as tetra-nucleotide frequency, of the query sequence.

At block 215, a target cluster is identified, based on the query vector, from amongst the plurality of reference clusters. For example, the cluster selection module 130 identifies the target cluster 165-1 from amongst the reference clusters 165 in the reference database 155. In one implementation, the reference database 155 may be a pre-configured database having pre-configured reference clusters 165. Alternately, the reference sequences may be classified into reference clusters 165 using the cluster selection module 130, as will be explained in detail with reference to description of Fig. 3.

In an implementation, the target cluster is identified based on the composition based analysis of the query sequence. For example, a non-Euclidean distance, such as Manhattan distance (L1 norm), may be computed between the query vector and the respective cluster centroid associated with each of the cluster in the reference database. Based on the computed distances, the target cluster may be selected. For example, a reference cluster having a minimum distance is selected as the target cluster 165-1.

At block 220, a similarity based analysis of the query sequence is performed with respect to reference sequences in the target cluster. For example, the assignment module 135 performs the similarity based analysis of the query sequence with the reference sequences 160-1A to 160-1Z, which are included in the target cluster 165-1. The assignment module 135 may implement conventional similarity based techniques, for example, BLAST, SOrt-ITEMS, etc. In one implementation, the query sequence may be translated into a corresponding amino acid query sequence. In such a case, the assignment module 135 compares the amino acid query sequence with the amino acid reference sequences corresponding to the reference sequences 160-1A to 160-1Z in the target cluster 165-1. Alternately, the query sequence, which may be in the form of a nucleic acid sequence, may be compared with nucleic acid reference sequences 160-1A to 160-1Z in the target cluster 165-1.

The comparison may provide information pertaining to the degree of homology between the query sequence and each of the reference sequences in the target cluster. The degree of homology may be based on the quality of alignment between the query sequence and each of the reference sequences in the target cluster. The quality of alignment may be computed based on one or more alignment parameters, such as bit-score, percentage of identity, and the percentage of positives between the query sequence and each of the reference sequences 160-1A to 160-1Z in the target cluster 165-1. In one implementation, the results of the comparison may be stored in analysis data, such as the analysis data 145, for further analysis.

At block 225, a taxon, from the target cluster, corresponding to the query sequence is identified and assigned to the query sequence. The taxon may be assigned to the query sequence based on the comparison done at block 220. Accordingly, one or more reference sequences illustrating a maximum degree of homology to the query sequence may be identified. Further, a taxon corresponding to these identified reference sequences may be assigned to the query sequence. For example, an assignment module, such as the assignment module 135, may identify and assign the taxon to the query sequence, based on the analysis data 145.

Since, in the present classification technique, the similarity-based analysis is performed with respect to the reference sequences 160-1A to 160-1Z in the target cluster 165-1 and not with respect to all the reference sequences 160 in the reference database 155, a reduction in the search space for the similarity-based analysis is achieved. The reduction in space, in turn, provides for substantial reduction in computing time and resources.

Referring to Fig. 3, the method 300 classifies a plurality of reference sequences, such as the reference sequences 160, into a plurality of reference clusters, such as the reference clusters 165, according to an implementation of the present subject matter. The reference sequences 160 may be classified using a cluster selection module, such as the cluster selection module 130, of the taxonomic classification system 100.

At block 305, reference sequences may be retrieved and saved in a reference database, such as the reference database 155. In one implementation, the taxonomic classification system may communicate with an external database, such as Genbank, and retrieve the reference sequences from the external database. The reference sequences may be retrieved in the form of nucleic acid sequences. These nucleic acid sequences may include either coding sequences or non-coding sequences or sequences including both coding and non-coding regions from partially or completely sequenced genomes. Additionally or alternately, the reference sequences may be retrieved in the form of amino acid sequences. Further, the reference database may contain reference sequences of prokaryotic organisms, eukaryotic organisms or both.

At block 310, it is determined whether the reference sequences are in the form of nucleic acid sequences or amino acid sequences. If the reference sequences are in the form of nucleic acid sequences, block 310 branches to block 315. Otherwise, block 310 directly proceeds to block 325.

At block 315, it is determined whether the reference database includes reference sequences containing both coding and non-coding regions from partially or completely sequenced genomes. If the reference database includes reference sequences containing both coding and non-coding regions, block 315 branches to block 320. Otherwise, block 315 directly branches to block 325.

At block 320, the reference sequences containing both coding and non-coding regions from partially or completely sequenced genomes are split into fragments of a predetermined length, for example, a fragment having 1000 base pairs and of the fragment sequence may be considered as a reference sequence. After fragmentation, block 320 branches to block 325.

At block 325, a reference vector corresponding to each of the reference sequences may be generated. Reference vectors may be generated based on one or more compositional characteristics of the reference sequences. For example, the reference vectors may be generated based on frequencies of all possible tetra-nucleotides in the reference sequence and the reference vectors may be represented in the form of 256 dimensional tetra-nucleotide frequency vectors.

At block 330, the reference sequences may be classified into reference clusters using a clustering technique, such as k-means clustering technique, based on the compositional characteristics of the reference vectors. In one implementation, the reference sequences with similar oligonucleotide composition may be classified together in one cluster.

At block 335, a cluster centroid may be assigned to each of the reference clusters. The cluster centroid may be computed based on the reference sequences that lie in that cluster. A cluster centroid represents the mean value of the reference vectors corresponding to the reference sequences included in a particular reference cluster.

At block 340, it is determined whether the translation of the reference sequences is required or not. If at block 340, it is determined that translation of the reference sequence is required, then block 340 branches to block 345. For example, if at block 310 it is determined that the reference sequences are in the form of nucleic acid sequences and the assignment module 135 involves comparison of the query sequence with the amino acid reference sequences, then block 340 branches to block 345, which subsequently branches to block 350.

If at block 340, it is determined that translation of the reference sequence is not required, then block 340 branches to block 350. For example, if at block 310 it is determined that the reference sequences are in the form nucleic acid reference sequences and the assignment module 135 involves comparison of the query sequence with the nucleic acid reference sequences, then block 340 branches to block 350. Similarly, if at block 310, it is determined that the reference sequences are in the form of amino acid reference sequences and the assignment module 135 involves comparison of the query sequence with the amino acid reference sequences, then also block 340 branches to block 350.

At block 345, the reference sequences are translated into corresponding amino acid reference sequences. The reference sequences may be translated using a six-frame translation technique. The amino acid reference sequences are tagged to corresponding reference clusters, which contain respective reference sequences.

At block 350 the clusters having the reference sequences and the corresponding cluster centroids of each reference cluster are stored in a reference database.

The method 300 for obtaining reference clusters based on one or more reference sequences not only reduces the computational time, but also maintains accuracy and specificity of the present taxonomic classification technique comparable to the conventional similarity based taxonomic classification techniques.

Although embodiments for taxonomic classification of metagenomic sequences have been described in language specific to structural features and/or methods, it is to be understood that the invention is not necessarily limited to the specific features or methods described. Rather, the specific features and methods are disclosed as exemplary embodiments for taxonomic classification of metagenomic sequences.

## Claims

1. A computer-implemented method of identifying a taxon corresponding to a query amino acid or nucleic acid sequence, the method comprising:
clustering a plurality of reference sequences (160), based on at least one compositional characteristic of the plurality of reference sequences (160), into a plurality of reference clusters (165), where the plurality of reference sequences (160) are known amino acid or nucleic acid sequences;
generating a query vector corresponding to the query sequence based on the at least one compositional characteristics of the query sequence and selecting at least one target cluster from amongst the plurality of reference clusters (165) corresponding to the query sequence, wherein the selecting is based on a composition-based analysis of the query sequence by computing a distance between the query vector and a corresponding cluster centroid associated with each of the reference clusters and selecting the at least one target cluster from amongst the plurality of reference clusters (165) based on a shortest distance from amongst the computed distances;
performing a similarity-based analysis of the query sequence with respect to one or more reference sequences in the at least one target cluster to determine a degree of homology between the query sequence and the plurality of reference sequences (160) in the at least one selected target cluster, wherein the degree of homology is computed based on one or more alignment parameters;
identifying one or more reference sequences illustrating maximum degree of homology to the query sequence; and
identifying a taxon corresponding to said reference sequences and assigning said taxon to the query sequence.

2. The method as claimed in claim 1, wherein the clustering comprises:
generating a plurality of reference vectors, indicative of the at least one compositional characteristic, corresponding to the plurality of reference sequences (160);
clustering the plurality of reference vectors into the plurality of reference clusters (165) based on the compositional characteristic of the reference vectors; and
assigning a cluster centroid (170) to each of the plurality of the reference clusters (165).

3. A taxonomic classification system (100) for identifying a taxon corresponding to a query amino acid or nucleic acid sequence, the system comprising:
a processor (110); and
a memory (115) coupled to the processor (110), the memory (115) comprising:
a cluster selection module (130) configured to:
cluster a plurality of reference sequences (160), based on at least one compositional characteristic of the plurality of reference sequences (160), into a plurality of reference clusters (165), where the plurality of reference sequences (160) are known amino acid or nucleic acid sequences; and
generate a query vector corresponding to the query sequence based on the at least one compositional characteristics of the query sequence and select at least one target cluster from amongst the plurality of reference clusters (165), corresponding to the query sequence, wherein the selecting is based on a composition-based analysis of the query sequence by computing a distance between the query vector and a corresponding cluster centroid associated with each of the reference clusters and selecting the at least one target cluster from amongst the plurality of reference clusters (165) based on a shortest distance from amongst the computed distances; and
an assignment module (135) configured to:
perform a similarity-based analysis of the query sequence with respect to one or more reference sequences in the at least one target cluster to determine a degree of homology between the query sequence and plurality of reference sequences (160) in the at least one selected target cluster, wherein the degree of homology is computed based on one or more alignment parameters;
identify one or more reference sequences illustrating maximum degree of homology to the query sequence; and
identify a taxon corresponding to said reference sequences and assign said taxon to the query sequence.

4. The taxonomic classification system as claimed in claim 3, wherein the reference sequences (160) are selected from the group consisting of coding sequences, non-coding sequences, and sequences including a combination of coding and non-coding regions derived from completely sequenced genomes.

5. The taxonomic classification system as claimed in claim 3, wherein the reference sequences (160) are derived from genomes of prokaryotic organisms.

6. The taxonomic classification system (100) as claimed in claim 3, wherein the cluster selection module (130) is further configured to:
generate a plurality of reference vectors, indicative of the at least one compositional characteristic, corresponding to the plurality of reference sequences (160);
cluster the plurality of reference vectors into the plurality of reference clusters (165) based on the at least one compositional characteristic of the plurality of reference vectors; and
assign a cluster centroid (170) to each of the plurality of the reference clusters (165).

7. A computer readable medium having computer executable instructions which when executed, implement a method of identifying a taxon corresponding to a query amino acid or nucleic acid sequence, the method comprising:
clustering a plurality of reference sequences (160), based on at least one compositional characteristic of the plurality of reference sequences (160), into a plurality of reference clusters (165), where the plurality of reference sequences (160) are known amino acid or nucleic acid sequences;
generating a query vector corresponding to the query sequence based on the at least one compositional characteristics of the query sequence and selecting at least one target cluster from amongst the plurality of reference clusters (165),corresponding to the query sequence, wherein the selecting is based on a composition-based analysis of the query sequence by computing a distance between the query vector and a corresponding cluster centroid associated with each of the reference clusters and selecting the at least one target cluster from amongst the plurality of reference clusters(165) based on a shortest distance from amongst the computed distances;
performing a similarity-based analysis of the query sequence with respect to one or more reference sequences in the at least one target cluster to determine a degree of homology between the query sequence and plurality of reference sequence (160) in the at least one selected target cluster, wherein the degree of homology is computed based on one or more alignment parameters; and
identifying one or more reference sequences illustrating maximum degree of homology to the query sequence; and
identifying a taxon corresponding to said reference sequences and assigning said taxon to the query sequence.

## Patentansprüche

1. Computerimplementiertes Verfahren zum Identifizieren eines einer Abfrageaminosäure- oder Nukleinsäuresequenz entsprechenden Taxons, wobei das Verfahren umfasst:
Ansammeln einer Vielzahl von Referenzsequenzen (160) auf Grundlage zumindest einer Zusammensetzungseigenschaft der Vielzahl von Referenzsequenzen (160), in eine Vielzahl von Referenzansammlungen (165), wobei die Vielzahl von Referenzsequenzen (160) bekannte Aminosäure- oder Nukleinsäuresequenzen sind;
Erzeugen eines der Abfragesequenz entsprechenden Abfragevektors auf Grundlage der zumindest einen Zusammensetzungseigenschaft der Abfragesequenz, und Auswählen zumindest einer Zielansammlung aus der der Abfragesequenz entsprechenden Vielzahl von Referenzansammlungen (165), wobei das Auswählen basiert ist auf einer zusammensetzungsbasierten Analyse der Abfragesequenz durch Berechnen eines Abstandes zwischen dem Abfragevektor und einem entsprechenden mit jeder der Referenzansammlungen verknüpften Ansammlungsschwerpunkt, und Auswählen der zumindest einen Zielansammlung aus der Vielzahl von Referenzansammlungen (165) auf Grundlage eines kürzesten Abstandes unter den berechneten Abständen;
Ausführen einer ähnlichkeitsbasierten Analyse der Abfragesequenz in Bezug auf eine oder mehrere Referenzsequenzen in der zumindest einen Zielansammlung, um einen Homologiegrad zwischen der Abfragesequenz und der Vielzahl von Referenzsequenzen (160) in der zumindest einen gewählten Zielansammlung zu bestimmen, wobei der Homologiegrad auf Grundlage von einem oder mehreren Angleichungsparametern berechnet wird;
Identifizieren einer oder mehrerer Referenzsequenzen, die den maximalen Homologiegrad an die Abfragesequenz illustrieren; und
Identifizieren eines den Referenzsequenzen entsprechenden Taxons, und Zuweisen des Taxons an die Abfragesequenz.

2. Verfahren nach Anspruch 1, wobei das Ansammeln umfasst:
Erzeugen einer Vielzahl von Referenzvektoren, die die mindestens eine Zusammensetzungseigenschaft angeben, entsprechend der Vielzahl von Referenzsequenzen (160);
Ansammeln der Vielzahl von Referenzvektoren in die Vielzahl von Referenzansammlungen (165) auf Grundlage der Zusammensetzungseigenschaft der Referenzvektoren; und
Zuweisen eines Ansammlungsschwerpunktes (170) an jede der Vielzahl von Referenzansammlungen (165).

3. Taxonomisches Klassifikationssystem (100) zum Identifizieren eines einer Abfrageaminosäure- oder Nukleinsäuresequenz entsprechenden Taxons, wobei das System umfasst:
einen Prozessor (110); und
einen Speicher (115), der mit dem Prozessor (110) verbunden ist, wobei der Speicher (115) umfasst:
ein Ansammlungsauswahlmodul (130) ausgelegt zum:
Ansammeln einer Vielzahl von Referenzsequenzen (160) auf Grundlage zumindest einer Zusammensetzungseigenschaft der Vielzahl von Referenzsequenzen (160), in eine Vielzahl von Referenzansammlungen (165), wobei die Vielzahl von Referenzsequenzen (160) bekannte Aminosäure- oder Nukleinsäuresequenzen sind; und
Erzeugen eines der Abfragesequenz entsprechenden Abfragevektors auf Grundlage der zumindest einen Zusammensetzungseigenschaft der Abfragesequenz, und Auswählen zumindest einer Zielansammlung aus der der Abfragesequenz entsprechenden Vielzahl von Referenzansammlungen (165), wobei das Auswählen basiert ist auf einer zusammensetzungsbasierten Analyse der Abfragesequenz durch Berechnen eines Abstandes zwischen dem Abfragevektor und einem entsprechenden mit jeder der Referenzansammlungen verknüpften Ansammlungsschwerpunkt, und Auswählen der zumindest einen Zielansammlung aus der Vielzahl von Referenzansammlungen (165) auf Grundlage eines kürzesten Abstandes unter den berechneten Abständen; und
ein Zuweisungsmodul (135), ausgelegt zum:
Ausführen einer ähnlichkeitsbasierten Analyse der Abfragesequenz in Bezug auf eine oder mehrere Referenzsequenzen in der zumindest einen Zielansammlung, um einen Homologiegrad zwischen der Abfragesequenz und Vielzahl von Referenzsequenzen (160) in der zumindest einen gewählten Zielansammlung zu bestimmen, wobei der Homologiegrad auf Grundlage von einem oder mehreren Angleichungsparametern berechnet wird;
Identifizieren einer oder mehrerer Referenzsequenzen, die den maximalen Homologiegrad an die Abfragesequenz illustrieren; und
Identifizieren eines den Referenzsequenzen entsprechenden Taxons, und Zuweisen des Taxons an die Abfragesequenz.

4. Taxonomisches Klassifikationssystem nach Anspruch 3, wobei die Referenzsequenzen (160) ausgewählt sind aus der Gruppe bestehend aus kodierenden Sequenzen, nicht-kodierenden Sequenzen und Sequenzen, die eine Kombination von kodierenden und nicht-kodierenden Bereichen, die von vollständig sequenzierten Genomen abgeleitet sind, enthalten.

5. Taxonomisches Klassifikationssystem nach Anspruch 3, wobei die Referenzsequenzen (160) abgeleitet sind von Genomen von prokaryotischen Organismen.

6. Taxonomisches Klassifikationssystem (100) nach Anspruch 3, wobei das Ansammlungsauswahlmodul (130) ferner ausgelegt ist, zum:
Erzeugen einer Vielzahl von Referenzvektoren, die die mindestens eine Zusammensetzungseigenschaft angeben, entsprechend der Vielzahl von Referenzsequenzen (160);
Ansammeln der Vielzahl von Referenzvektoren in die Vielzahl von Referenzansammlungen (165) auf Grundlage der mindestens einen Zusammensetzungseigenschaft der Referenzvektoren; und
Zuweisen eines Ansammlungsschwerpunktes (170) an jede der Vielzahl von Referenzansammlungen (165).

7. Computerlesbares Medium mit computerausführbaren Anweisungen, die beim Ausführen ein Verfahren zum Identifizieren eines einer Abfrageaminosäure- oder Nukleinsäuresequenz entsprechenden Taxons implementieren, wobei das Verfahren umfasst:
Ansammeln einer Vielzahl von Referenzsequenzen (160) auf Grundlage zumindest einer Zusammensetzungseigenschaft der Vielzahl von Referenzsequenzen (160), in eine Vielzahl von Referenzansammlungen (165), wobei die Vielzahl von Referenzsequenzen (160) bekannte Aminosäure- oder Nukleinsäuresequenzen sind;
Erzeugen eines der Abfragesequenz entsprechenden Abfragevektors auf Grundlage der zumindest einen Zusammensetzungseigenschaft der Abfragesequenz, und Auswählen zumindest einer Zielansammlung aus der der Abfragesequenz entsprechenden Vielzahl von Referenzansammlungen (165), wobei das Auswählen basiert ist auf einer zusammensetzungsbasierten Analyse der Abfragesequenz durch Berechnen eines Abstandes zwischen dem Abfragevektor und einem entsprechenden mit jeder der Referenzansammlungen verknüpften Ansammlungsschwerpunkt, und Auswählen der zumindest einen Zielansammlung aus der Vielzahl von Referenzansammlungen (165) auf Grundlage eines kürzesten Abstandes unter den berechneten Abständen;
Ausführen einer ähnlichkeitsbasierten Analyse der Abfragesequenz in Bezug auf eine oder mehrere Referenzsequenzen in der zumindest einen Zielansammlung, um einen Homologiegrad zwischen der Abfragesequenz und Vielzahl von Referenzsequenz (160) in der zumindest einen gewählten Zielansammlung zu bestimmen, wobei der Homologiegrad auf Grundlage von einem oder mehreren Angleichungsparametern berechnet wird; und
Identifizieren einer oder mehrerer Referenzsequenzen, die den maximalen Homologiegrad an die Abfragesequenz illustrieren; und
Identifizieren eines den Referenzsequenzen entsprechenden Taxons, und Zuweisen des Taxons an die Abfragesequenz.

## Revendications

1. Procédé exécuté sur ordinateur et destiné à l'identification d'un taxon correspondant à une séquence de requête d'acide aminé ou d'acide nucléique, le procédé comprenant :
le regroupement d'une pluralité de séquences de référence (160), sur la base d'au moins une caractéristique de composition de la pluralité de séquences de référence (160), dans une pluralité de regroupements de référence (165), la pluralité de regroupements de référence (160) étant des séquences connues d'acide aminé ou d'acide nucléique ;
la génération d'un vecteur de requête correspondant à la séquence de requête sur la base de l'au moins une caractéristique de composition de la séquence de requête et la sélection d'au moins un regroupement cible parmi la pluralité de groupements de référence (165) correspondant à la séquence de requête, la sélection étant basée sur une analyse basée sur la composition de la séquence de requête en calculant une distance entre le vecteur de requête et un centroïde de regroupement correspondant associé avec chacun des regroupements de référence et sélectionnant l'au moins un regroupement cible parmi la pluralité de groupements de référence (165) sur la base d'une distance la plus courte parmi les distances calculées ;
l'exécution d'une analyse basée sur la similarité de la séquence de requête par rapport à l'une ou plusieurs séquences de référence dans l'au moins un regroupement cible pour déterminer un degré d'homologie entre la séquence de requête et la pluralité de séquences de référence (160) dans l'au moins un regroupement cible sélectionné, le degré d'homologie pouvant être calculé sur la base d'un ou plusieurs paramètres d'alignement ;
l'identification d'une ou de plusieurs séquences de référence illustrant le degré maximal d'homologie à la séquence de requête ; et
l'identification d'un taxon correspondant auxdites séquences de référence et l'affectation dudit taxon à ladite séquence de requête.

2. Procédé selon la revendication 1, dans lequel le regroupement comprend :
la génération d'une pluralité de vecteurs de référence, indicatifs de l'au moins une caractéristique de composition, correspondant à la pluralité de séquences de référence (160) ;
le regroupement de la pluralité de vecteurs de référence dans la pluralité de regroupements de référence (165) sur la base de la caractéristique de composition des vecteurs de référence ; et
l'affectation d'un centroïde de regroupement (170) à chacun de la pluralité des regroupements de référence (165).

3. Système de classification taxonomique (100) pour l'identification d'un taxon correspondant à une séquence de requête d'acide aminé ou d'acide nucléique, le système comprenant :
un processeur (110) ; et
une mémoire (115) couplée au processeur (110), la mémoire (115) comprenant :
un module de sélection de regroupement (130) configuré pour :
le regroupement d'une pluralité de séquences de référence (160), sur la base d'au moins une caractéristique de composition de la pluralité de séquences de référence (160), dans une pluralité de regroupements de référence (165), la pluralité de regroupements de référence (160) étant des séquences connues d'acide aminé ou d'acide nucléique ;
la génération d'un vecteur de requête correspondant à la séquence de requête sur la base d'au moins une caractéristique de composition de la séquence de requête et la sélection d'au moins un regroupement cible parmi la pluralité de groupements de référence (165) correspondant à la séquence de requête, la sélection étant basée sur une analyse basée sur la composition de la séquence de requête en calculant une distance entre le vecteur de requête et un centroïde de regroupement correspondant associé avec chacun des regroupements de référence et sélectionnant l'au moins un regroupement cible parmi la pluralité de groupements de référence (165) sur la base d'une distance la plus courte parmi les distances calculées ;
un module d'affectation de taxon (135) configuré pour :
l'exécution d'une analyse basée sur la similarité de la séquence de requête par rapport à l'une ou plusieurs séquences de référence dans l'au moins un regroupement cible pour déterminer un degré d'homologie entre la séquence de requête et la pluralité de séquences de référence (160) dans l'au moins un regroupement cible sélectionné, le degré d'homologie étant calculé sur la base d'un ou de plusieurs paramètres d'alignement ;
l'identification d'une ou de plusieurs séquences de référence illustrant le degré maximal d'homologie à la séquence de requête ; et
l'identification d'un taxon correspondant auxdites séquences de références et l'affectation dudit taxon à ladite séquence de requête.

4. Système de classification taxonomique selon la revendication 3, dans lequel les séquences de référence (160) sont sélectionnées parmi le groupe constitué de séquences codantes, de séquences non codantes et de séquences comprenant une combinaison de régions codantes et non codantes dérivées de génomes complètement séquencés.

5. Système de classification taxonomique selon la revendication 3, dans lequel les séquences de référence (160) sont dérivées de génomes d'organismes procaryotes.

6. Système de classification taxonomique (100) selon la revendication 3, dans lequel le module de sélection de taxon (130) est en outre configuré pour :
la génération d'une pluralité de vecteurs de référence, indicatifs de l'au moins une caractéristique de composition, correspondant à la pluralité de séquences de référence (160) ;
le regroupement de la pluralité de vecteurs de référence dans la pluralité de regroupements de référence (165) sur la base de l'au moins une caractéristique de composition de la pluralité de vecteurs de référence ; et
l'attribution d'un centroïde de regroupement (170) à chacun de la pluralité des regroupements de référence (165).

7. Support lisible par ordinateur ayant des instructions exécutables par ordinateur qui, lorsqu'elles sont exécutées, implémentent un procédé d'identification d'un taxon correspondant à une séquence de requête d'acide nucléique ou d'acide aminé, le procédé comprenant :
le regroupement d'une pluralité de séquences de référence (160), sur la base d'au moins une caractéristique de composition de la pluralité de séquences de référence (160), dans une pluralité de regroupements de référence (165), la pluralité de séquences de référence (160) étant des séquences connues d'acide aminé ou d'acide nucléique ;
la génération d'un vecteur de requête correspondant à la séquence de requête sur la base de l'au moins une caractéristique de composition de la séquence de requête et la sélection d'au moins un regroupement cible parmi la pluralité de groupements de référence (165) correspondant à la séquence de requête, la sélection étant basée sur une analyse basée sur la composition de la séquence de requête en calculant une distance entre le vecteur de requête et un centroïde de regroupement correspondant associé avec chacun des regroupements de référence et sélectionnant l'au moins un regroupement cible parmi la pluralité de groupements de référence (165) sur la base d'une distance la plus courte parmi les distances calculées ;
l'exécution d'une analyse basée sur la similarité de la séquence de requête par rapport à l'une ou plusieurs séquences de référence dans l'au moins un regroupement cible pour déterminer un degré d'homologie entre la séquence de requête et la pluralité de séquences de référence (160) dans l'au moins un regroupement cible sélectionné, le degré d'homologie étant calculé sur la base d'un ou plusieurs paramètres d'alignement ; et
l'identification d'une ou de plusieurs séquences de référence illustrant le degré maximal d'homologie à la séquence de requête ; et
l'identification d'un taxon correspondant auxdites séquences de références et l'affectation dudit taxon à ladite séquence de requête.
